# EUROPÄISCHE PATENTSCHRIFT

(11) **EP 0 613 461 B1**
(45) Veröffentlichungstag und Bekanntmachung des Hinweises auf die Patenterteilung: **01.05.1996**
(21) Anmeldenummer: 92922850.0
(22) Anmeldetag: 04.11.1992
(51) Int. Cl.: C07C 303/24, C07C 305/06

(54) **VERFAHREN ZUR HERSTELLUNG SULFATIERTER FETTSÄUREALKANOLAMIDE**
METHOD OF PREPARING SULPHATED FATTY-ACID ALKANOLAMIDES
PROCEDE DE FABRICATION D'ALCANOLAMIDES D'ACIDES GRAS SULFATES

(30) Priorität: 13.11.1991 DE 4137221
(43) Veröffentlichungstag der Anmeldung: 07.09.1994
(73) Patentinhaber: Henkel Kommanditgesellschaft auf Aktien, D-40191 Düsseldorf (DE)
(72) Erfinder: BEHLER, Ansgar, D-4250 Bottrop (DE); PLOOG, Uwe, D-5657 Haan (DE); CLASEN, Frank, D-4010 Hilden (DE)
(86) Internationale Anmeldenummer: EP9202527
(87) Internationale Veröffentlichungsnummer: WO9310084

(56) Entgegenhaltungen:
- DE-A- 3 906 096
- DE-B- 1 195 301
- NL-B- 125 242
- CHEMICAL ABSTRACTS, vol. 98, no. 15, 11. April 1983, Columbus, Ohio, US; abstract no. 125448g, V.T. EFIMOV ET AL. 'Sulfatized alkylolamides of fatty acids.' Seite 582 ;Spalte 1 ; & SU, A,979 334

## Beschreibung

### Gebiet der Erfindung

Die Erfindung betrifft ein Verfahren zur Herstellung sulfatierter Fettsäurealkanolamide durch kontinuierliche Sulfatierung von Fettsäurealkanolamiden mit gasförmigem Schwefeltrioxid und Neutralisation der sauren Reaktionsprodukte mit wäßrigen Basen.

### Stand der Technik

Sulfate von Fettsäurealkanolamiden stellen anionische Tenside dar, die über ein ausgeprägtes Schaumvermögen und geringe Härteempfindlichkeit verfügen und daher Anwendung z. B. in kosmetischen Produkten finden. Zu ihrer Herstellung geht man üblicherweise von Fettsäuren, Fettsäureestern oder Fettsäurechloriden aus, die zunächst mit Alkanolaminen amidiert und anschließend mit geeigneten Sulfiermitteln sulfatiert werden.

Aus **J.Rech.Centre Natl.Rec.Sci., 13, 169 (1950),** zitiert nach **Chem.Abstr., 45, 10617 (1951)** und **J.Am.Chem.Soc., 47, 91 (1970)** ist bekannt, daß sich Fettsäurealkanolamide diskontinuierlich mit Chlorsulfonsäure in Chloroform oder Tetrachlorkohlenstoff sulfatieren lassen.

Aus der **US 1,981,792** ist des weiteren ein Verfahren zur Herstellung von Fettsäurealkanolamidaulfaten bekannt, bei dem die Sulfatierung diskontinuierlich mit Schwefelsäure, gegebenenfalls unter Verwendung von chlorierten Lösungsmitteln durchgeführt wird.

Gemäß der Lehre der **US 2,588,197** können Fettsäurealkanolamide auch diskontinuierlich mit chlorsulfonsäure in Schwefelsäure oder Dichlormethan als Lösungsmittel sulfatiert werden.

In **J.Am.Ch-.Soc., 73, 3642 (1951)** wird die Umsetzung von N-Acylmyristamid mit Schwefelsäure beschrieben, die geringe Mengen gelöstes Schwefeltrioxid enthält. Bei Steigerung des SO₃-Gehaltes auf 22 Gew.-% werden jedoch nur noch dunkel verfärbte Produkte erhalten.

In **J.Am.Oil.Chem.Soc. 54, 371 (1977)** wird schließlich über die Umsetzung von Talgisopropanolamid mit Chlorsulfonsäure bei 55 bis 60°C berichtet. Die resultierenden Sulfatierungsprodukte sind jedoch hochviskos und dunkel verfärbt. Eine Erhöhung der Reaktionstemperatur zur Erniedrigung der Viskosität führt zu einer Verkohlung der Proukte. Gemäß der **US 4,116,986** wird in diesem Zusammenhang vorgechlagen, die Sulfatierung in Gegenwart eines niedermolekularen Alkohols durchzuführen.

Aus **Chem.Abstr., Vol.98, Nr.15, 1983, S.582, Abstr.Nr. 125448g** ist die Sulfatierung von C₆-C₂₀-Fettsäurealkanolamiden mit einem SO₃/Inertgasgemisch sowie die Neutralisation der Sulfatierungsprodukte bekannt. Die Schrift enthält keinen Hinweis auf den Sulfierreaktor oder eine Nachreaktion.

In der Deutschen Patentanmeldung **DE-A 1195301** wird ein Verfahren zur Sulfierung von Fettstoffen, darunter auch Fettsäureethanolamiden, vorgeschlagen, bei dem man die Einsatzstoffe unter Überdruck in einer Mischkammer verwirbelt und gemeinsam in eine Austragskammer versprüht.

Aus der Niederländischen Patentanmeldung **NL-A 125242** ist schließlich ebenfalls ein Verfahren zur Sulfatierung von Fettsäurealkanolamiden unter Einsatz von SO₃/Inertgas-Gemischen bekannt, wobei ein Reaktor vorgeschlagen wird, bei dem das Reaktionsrohr in Art einer Siebbodenkolonne ausgelegt ist. Der erhitzte Strom der Ausgangsmaterialien tropft über die durchbohrten Bodenplatten bis in die Schleusenkammer und wird dabei in mehreren Stufen begast.

Die Aufgabe der Erfindung bestand somit darin, ein verbessertes Verfahren zur Herstellung von sulfatierten Fettsäurealkanolamiden zu entwickeln, das frei von den geschilderten Nachteilen ist.

### Beschreibung der Erfindung

Gegenstand der Erfindung ist ein Verfahren zur Herstellung sulfatierter Fettsäurealkanolamide, das sich dadurch auszeichnet, daß man a) Fettsäurealkanolamide kontinuierlich mit gasförmigem Schwefeltrioxid in einem Reaktor umseht, der nach dem Fallfilmprinzip arbeitet, b) die sauren Reaktionsprodukte anschließend mit wäßrigen Basen neutralisiert und c) danach über einen Zeilraum von 0,1 bis 1 h bei einer Temperatur von 70 bis 90°C altert, wobei der pH-Wert auf 6 bis 8 gehalten wird.

Überraschenderweise wurde gefunden, daß sich Fettsäurealkanolamide ohne Viskositätsprobleme in kontinuierlichen Reaktoren mit gasförmigem Schwefeltrioxid umsetzen lassen. Trotz der vergleichsweise hohen Sulfiertemperatur sind die Produkte zudem auch ohne Bleiche unerwartet hellfarbig.

Unter Fettsäurealkanolamide sind Stoffe der Formel (I) zu verstehen, 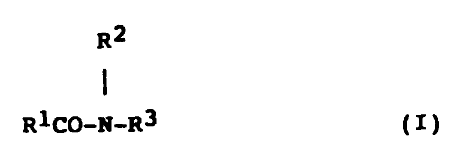 in der
- R¹CO: für einen linearen oder verzweigten, aliphatischen Acylrest mit 0, 1, 2 oder 3 Doppelbindungen,
- R: für Wasserstoff oder einen Hydroxyalkylrest mit 2 bis 6 Kohlenstoffatomen und
- R³: für einen Hydroxyalkylrest mit 2 bis 6 Kohlenstoffatomen
steht.

Typische Beispiele sind die Monoethanolamide, Mono-n-propanolamide, Mono-i-propanolamide, Monobutanolamide, Monopentanolamide, Monohexanolamide, Diethanolamide, Di-n-propanolamide und Di-i-propanolamide der Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Arachinsäure, Gadoleinsäure, Behensäure und Erucasäure. Bevorzugte Einsatzstoffe sind die Monoethanol- und Monopropanolamide von gesättigten Fettsäuren mit 8 bis 18 Kohlenstoffatomen.

Wie in der Fettchemie üblich, kann sich die Fettsäurekomponente der Amide auch von technischen Fettsäureschnitten ableiten, wie sie bei der Druckspaltung von natürlichen Fetten und Ölen, beispielsweise, Palmöl, Palmkernöl, Kokosöl, Rüböl oder Rindertalg anfallen. Bevorzugte Einsatzstoffe sind die Monoethanol- und Monopropanolamide von Kokos- bzw. Talgfettsäuren mit 12 bis 18 Kohlenstoffatomen.

Die kontinuierliche Sulfatierung der Fettsäurealkanolamide mit gasförmigem Schwefeltrioxid kann in der für Fettsäureniedrigalkylester bekannten Weise **[J.Falbe (ed.), "Surfactants in consumer products", Springer-Verlag, Berlin-Heidelberg, 1987, S.61]** erfolgen, wobei Reaktoren, die nach dem Fallfilmprinzip arbeiten, zur Herstellung besonders hellfarbiger Produkt eingeseht werden. Dabei wird das Schwefeltrioxid mit Luft oder Stickstoff verdünnt und in Form eines Gasgemisches, welches das SO₃ in einer Konzentration von 1 bis 8, vorzugsweise 3 bis 5 Vol.-% enthält, eingesetzt.

Das molare Einsatzverhältnis von Fettsäurealkanolamid zu Schwefeltrioxid kann 1 : 0,95 bis 1 : 2,0 betragen. Werden Fettsäuremonoalkanolamide mit einer freien Hydroxylgruppe eingesetzt, hat es sich als optimal erwiesen, ein Einsatzverhältnis von 1 : 1 bis 1 : 1,3 zu wählen. Für die Sulfatierung von Fettsäuredialkanolamiden, die über zwei freie Hydroxylgruppen verfügen, ist es empfehlenswert, höhere Einsatzmengen, beispielsweise ein molares Verhältnis von 1 : 1,3 bis 1 : 1,8 einzustellen.

Die Sulfatierung kann bei einer Temperatur von 80 bis 95°C durchgeführt werden. Im Hinblick auf einen möglichst hohen Umsetzungsgrad hat sich ein Temperaturbereich von 90 bis 95°C als besonders vorteilhaft erwiesen.

Bei der Umsetzung der Fettsäurealkanolamide mit Schwefeltrioxid findet eine Sulfatierung der freien Hydroxylgruppen unter Bildung von sauren Schwefelsäurehalbestern statt. In untergeordneten Mengen können auch alpha-Fettsäureamidsulfonate sowie im Falle der Verwendung ungesättigter Fettsäurealkanolamide als Einsatzstoffe, Additionsprodukte des SO₃ an die Doppelbindung entstehen.

Im Anschluß an die Sulfatierung werden die sauren Sulfierprodukte in wäßrige Basen eingerührt und neutralisiert. Dieser Vorgang kann diskontinuierlich erfolgen, vorzugsweise wird jedoch kontinuierlich gearbeitet. Als Basen für die Neutralisation kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine in Betracht. Die Neutralisationsbasen gelangen dabei vorzugsweise in Form 5 bis 55 gew.-%iger wäßriger Lösungen zum Einsatz, wobei 5 bis 25 gew.-%ige wäßrige Natriumhydroxidlösungen bevorzugt sind.

Nach der Neutralisation werden die Produkte über einen Zeitraum von 0,1 bis 1 h bei einer Temperatur von 70 bis 90°C gealtert, wobei der pH-Wert bei 6 bis 8 gehalten wird. Bei kontinuierlicher Fahrweise empfiehlt es sich, die Neutralisation bei erhöhter Temperatur durchzuführen und die Alterung gegebenenfalls in einer Verweilzeitschlange vorzunehmen. Die sulfatierten Fettsäurealkanolamide fallen im Verlauf der Neutralisation als wäßrige Pasten mit einem Feststoffgehalt von 10 bis 70, vorzugsweise 25 bis 35 Gew.-% - bezogen auf die Paste - an.

Die Farbe der Sulfatierungsprodukte kann - falls dies gewünscht wird - nach der Neutralisation in an sich bekannter Weise durch Zusatz von Wasserstoffperoxid- und/oder Natriumhypochloritlösung weiter aufgehellt werden. Dabei werden - bezogen auf den Feststoffgehalt in der Lösung der Sulfatierungsprodukte - 0.2 bis 2 Gew.-% Wasserstoffperoxid - berechnet als 100 %ige Substanz - und/oder entsprechende Mengen Natriumhypochlorit eingesetzt.

Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die nach dem erfindungsgemäßen Verfahren hergestellten Fettsäurealkanolamidsulfate sind in Form 10 bis 50 gew.-%iger wäßriger Pasten leichtbeweglich und ohne Bleiche hellfarbig. Sie weisen ein ausgeprägtes Schaumvermögen auf, sind wenig härteempfindlich und in Wasser klar löslich. Sie eignen sich daher zur Herstellung von Wasch-, Spül- und Reinigungsmitteln sowie Produkten zur Haar- und Körperpflege, in denen sie in Mengen von 0,1 bis 25, vorzugsweise 1 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiel 1:

**C**_{**8/18**}**-Kokosfettsäureisopropanolamid-sulfat-Ha-Salz**

1a) Amidierung von Kokosfettsäure
   In einem 2-1-Dreihalskolben mit Tropftrichter, Rührer, Innenthermometer und Destillationsaufsatz wurden 936 g (4,7 mol) technische Kokosfettsäure (Edenor^{(R)} HK8-18, Fa.Henkel KGaA, Düsseldorf, FRG) unter Stickstoffatmosphäre auf 80°C erhitzt. Anschließend wurden innerhalb von 30 min 353 g (4,7 mol) Isopropanolamin zugetropft. Nach der Zugabe wurde die Reaktionsmischung weitere 2,5 h auf 180°C erhitzt, wobei 83 ml Wasser abdestilliert wurden. Das Produkt wies eine Aminzahl von 9,4 und eine Säurezahl von 6,5 auf.
1b) Sulfatierung
   In einem Kontinuierlich arbeitenden Fallfilmreaktor (länge 120 cm, Querschnitt 1 cm, Eduktdurchsalz 600 g/h) mit Mantelkühlung und sertlicher SO₃ - Begasung wurden 512 (2 mol) Kokosfettsäureisopropanolamid aus 1a) mit 168 g (2,1 mol) Schwefeltrioxid - entsprechend einem molaren Einsatzverhältnis von 1 : 1,05 - umgesetz. Das saure Sulfierprodukt wurde kontinuierlich in eine 37 Gew.%ige wäßrige Natriumhydroxidlösung emgetragen und neutralisiert. Nach der Neutralisation wurde das Produkt über einen Zeitraum von ca. 1 h bei 80 bis 95°C gealtert, wobei durch portionsweise Zugabe von wäßriger Natriumhydroxidlösung der pH zwischen 6 und 8 gehalten wurde.

### Kenndaten des Produktes:

| | |
|---|---|
| Aniontensidgehalt : | 23,4 Gew.-% |
| Unsulfierte Anteile : | 3,6 Gew.-% |
| Sulfat : | 3,7 Gew.-% |
| Wasser : | 69,3 Gew.-% |

### Beispiel 2:

**C**_{**12/14**}**-Kokosfettsäureisopropanolamid-sulfat-Na-Salz**

2a) Amidierung von Kokosfettsäure
Analog Beispiel 1a) wurden 941 g (4,5 mol) eines Kokosfettsäureschnittes (Edenor^{(R)} C₁₂-70, Fa.Henkel KGaA, Düsseldorf, FRG) mit 338 g (4,5 mol) Isopropanolamin umgesetzt. Das Reaktionsprodukt wies eine Aminzahl von 9,3 und eine Säurezahl von 11 auf.
2b) Sulfatierung
Analog Beispiel 1b) wurden 532 g (2 mol) des Kokosfettsäureamids aus 2a) mit 170 g (2,12 mol) - entsprechend einem molaren Einsatzverhältnis von 1 : 1,06 - umgesetzt.

| | |
|---|---|
| Aniontensidgehalt : | 17,4 Gew.-% |
| Unsulfierte Anteile : | 3,5 Gew.-% |
| Sulfat : | 3,5 Gew.-% |
| Wasser : | 75,6 Gew.-% |

Der Aniontensidgehalt (WAS) und die unsulfierten Anteile (US) wurden nach den DGF-Einheitsmethoden, Stuttgart 1950-1984, H-III-10 und G-II-6b ermittelt. Der Sulfatgehalt wurde als Natriumsulfat berechnet und das Wasser nach der Fischer-Methode bestimmt.

## Patentansprüche

1. Verfahren zur Herstellung sulfatierter Fettsäurealkanolamide, bei dem man
a) Fettsäurealkanolamide kontiuierlich mit gasförmigem Schwefeltrioxid in einem Reaktor umsetzt, der nach dem Fallfilmprinzip arbeitet,
b) die sauren Reaktionsprodukte anschließend mit wäßrigen Basen neutralisiert und
c) danach über einen Zeitraum von 0,1 bis 1 h bei einer Temperatur von 70 bis 90°C altert, wobei der pH-Wert auf 6 bis 8 gehalten wird.

2. Verfahren nach Anspruch 1, **dadurch gekennzeichnet**, daß man Fettsäurealkanolamide der Formel (I) einsetzt, 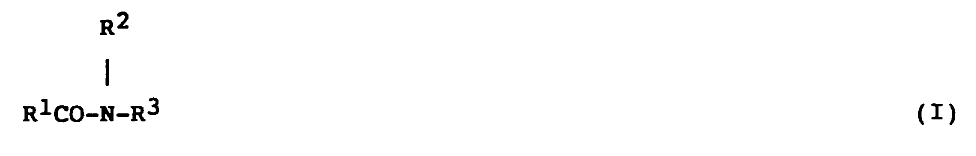 in der R¹CO für einen linearen oder verzweigten, aliphatischen Acylrest mit 0, 1, 2 oder 3 Doppelbindungen, R für Wasserstoff oder einen Hydroxyalkylrest mit 2 bis 6 Kohlenstoffatomen und R³ für einen Hydroxyalkylrest mit 2 bis 6 Kohlenstoffatomen steht.

3. Verfahren nach den Ansprüchen 1 und 2, **dadurch gekennzeichnet,** daß man das Schwefeltrioxid mit Luft oder Stickstoff auf eine Konzentration von 1 bis 8 Vol.-% - bezogen auf die Mischung - verdünnt.

4. Verfahren nach den Ansprüchen 1 bis 3, **dadurch gekennzeichnet,** daß man die Fettsäurealkanolamide und das Schwefeltrioxid in einem molaren Verhältnis von 1 : 0,95 bis 1 : 2,0 einsetzt.

5. Verfahren nach den Ansprüchen 1 bis 4, **dadurch gekennzeichnet,** daß man die Sulfatierung bei einer Temperatur von 80 bis 98°C durchführt.

6. Verfahren nach den Ansprüchen 1 bis 5, **dadurch gekennzeichnet,** daß man die Neutralisation mit 5 bis 55 gew.-%igen wäßrigen Basen aus der von Alkalimetallhydroxiden, Erdalkalimetalloxiden und -hydroxiden, Ammoniak, Mono-, Di- und Tri-C₂₋₄-Alkanolaminen sowie primären, sekundären und tertiären C₁₋₄-Alkylaminen gebildeten Gruppe durchführt.

## Claims

1. A process for the production of sulfated fatty acid alkanolamides, in which
a) fatty acid alkanolamides are continuously reacted with gaseous sulfur trioxide in a reactor operating on the falling film principle,
b) the acidic reaction products are subsequently neutralized with aqueous bases and
c) are then aged for 0.1 to 1 h at a temperature of 70 to 90°C, the pH being kept at a value of 6 to 8.

2. A process as claimed in claim 1, **characterized in that** fatty acid alkanolamides corresponding to formula (I): 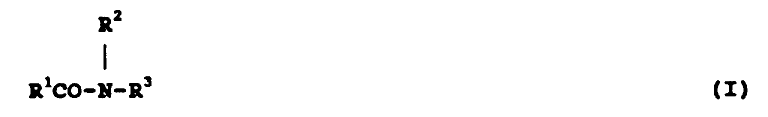 in which
R¹CO is a linear or branched, aliphatic acyl radical containing 0, 1, 2 or 3 double bonds, R is hydrogen or a hydroxyalkyl radical containing 2 to 6 carbon atoms and R³ is a hydroxyalkyl radical containing 2 to 6 carbon atoms, are used.

3. A process as claimed in claims 1 and 2, **characterized in that** the sulfur trioxide is diluted with air or nitrogen to a concentration of 1 to 8% by volume, based on the mixture.

4. A process as claimed in claims 1 to 3, **characterized in that** the fatty acid alkanolamides and the sulfur trioxide are used in a molar ratio of 1:0.95 to 1:2.0.

5. A process as claimed in claims 1 to 4, **characterized in that** the sulfation is carried out at a temperature of 80 to 98°C.

6. A process as claimed in claims 1 to 5, **characterised in that** the neutralization is carried out with 5 to 55% by weight aqueous bases from the group consisting of alkali metal hydroxides, alkaline earth metal oxides and hydroxides, ammonia, mono-, di- and tri-C₂₋₄-alkanolamines and also primary, secondary and tertiary C₁₋₄ alkylamines.

## Revendications

1. Procédé de préparation d'alcanolamide d'acides gras sulfatés dans lequel :
a) on fait réagir en continu des alcanolamides d'acides gras avec du trioxyde de soufre dans un réacteur, qui fonctionne selon le principe du ruissellement,
b) on neutralise les produits de réaction acides ensuite avec des bases aqueuses et,
c) ensuite on fait mûrir pendant une période de 0,1 à 1 heure à une température de 70 à 90°C, en maintenant le pH entre 6 et 8.

2. Procédé selon la revendication 1, caractérisé en ce qu'on utilise des alcanolamides d'acide gras de formule (I) 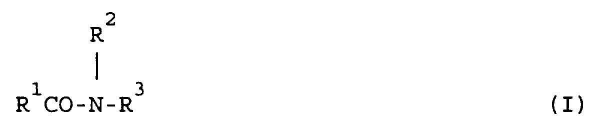
où R¹CO représente un reste acyle aliphatique, linéaire ou ramifié ayant 0,1,2 ou 3 doubles liaisons,
R est l'hydrogène ou un reste hydroxyalkyle ayant 2 à 6 atomes de carbone et,
R³ est un reste hydroxyalkyle ayant de 2 à 6 atomes de carbone.

3. Procédé selon les revendications 1 et 21, caractérisé en ce qu'on dilue le trioxyde de soufre avec de l'air ou de l'azote à une concentration de 1 à 8 % en volume par rapport au mélange.

4. Procédé selon les revendications 1 à 3, caractérisé en ce qu'on utilise les alcanolamides d'acides gras et le trioxyde de soufre en un rapport molaire de 1:0,95 à 1:2,0.

5. Procédé selon les revendications 1 à 4, caractérisé en ce qu'on réalise la sulfatation à une température de 80 à 98°C.

6. Procédé selon les revendications 1 à 5, caractérisé en ce qu'on réalise la neutralisation avec des solutions aqueuses de bases de 5 à 55 % en poids choisies dans le groupe constitué des hydroxydes de métaux alcalins, oxydes et hydroxydes de métaux alcalino-terreux, ammoniac, mono-, di- et tri(alcanol C₂₋₄)amines ainsi que des (alkyl C₁₋₄)amines primaires, secondaires et tertiaires.
